# EUROPEAN PATENT APPLICATION

(11) **EP 4 647 106 A1**
(43) Date of publication of application: **12.11.2025**
(21) Application number: 24193632.7
(22) Date of filing: 08.08.2024
(51) Int. Cl.: A61M 31/00

(54) **INTRAURETHRAL ADMINISTRATION DEVICE AND KIT**

(30) Priority: 07.05.2024 BR 102024008972
(71) Applicant: Instituto Galzu - Pesquisa, Ensino, Ciência e Tecnologia Aplicada, Campos dos Goytacazes, RJ (BR)
(72) Inventor: Jardim Guaré, Maria Regina, Sorocaba, SP (BR); Gil, Cristian Hugo, Buenos Aires (AR)
(74) Representative: Grünecker Patent- und Rechtsanwälte PartG mbB

(57) **Abstract**

Intraurethral administration of medications is a practice known for rapid absorption, reduced side effects and the toxicity, in addition to reduce the pharmaceutical formulations dose. Present invention aims ergonomics and comfort for using urethral device administration of liquid or gel medication directly into the man's urethral channel. More specifically, the device comprises a housing with domed shape associated to a non-standard syringe increased volumetric capacity and extended nozzle, plunger lock, cover and kit for accommodate the small intraurethral applicator. The present invention belongs to the fields of Pharmacy and Medicine and among the various applications, special emphasis is placed on erectile dysfunction, but this urethral route can also be used to variety of pharmaceutical formulations as: anti-inflammatories, antiseptics, antibiotics, anesthetics, among other applications with different color device, allowing the use both by the patient (self-administration) and by the healthcare professional.

## Description

### Field of the Invention

Present invention aims ergonomics and comfort for using urethral device administration of liquid or gel medication directly into the man's urethral channel. The present invention belongs to the fields of Pharmacy and Medicine.

### Background of the Invention

Intraurethral administration of medications is a practice known for rapid absorption, reduced side effects and toxicity, in addition a reduced the dosage of pharmaceutical formulations. Among the various applications, special emphasis is placed on erectile dysfunction, but this route can also be used for anti-inflammatories, antiseptics, antibiotics, anesthetics, among other applications. With this in mind, a small intraurethral applicator with a non-standard syringe with extended nozzle was developed.

Several devices have already been created, but eligibility for use still faces some technical difficulties.

As an example of this, the document WO9628142A1 discloses a transurethral administration device where a solid tablet is stored in a shaft that ejects it all at once via a piston when a lid is removed. For use, a plunger tip is pushed into a segment being connected to the shaft by means of an external flange. Although this solution of WO9628142A1 allows intraurethral administration, the device cannot be classified as a syringe, because this device ejects a solid tablet and lacks a graduation form for measuring the administration. Furthermore, the external flange fixes the shaft externally to segment, where the fingers are positioned, since the length of the shaft needs to be longer to store the medicine and increases the distance between the fingers and the application site of the device, hindering ergonomics and precision of application, not confusing with present invention.

Other example of this, document GB1321920A discloses a hollow device for introducing a solid suppository of cylindrical or globular shape into an anal or vaginal cavity. This device does not meet the requirements for intraurethral application, cannot be classified as a syringe and also does not apply liquid or gel, not confusing with present invention.

Other example of this, document WO2017082792A1 refers to a device for inserting a solid suppository comprising a body, a piston slidably inserted into the body, a tubular part defining a cavity and an upper section with an opening for receiving a suppository. This device does not meet the requirements for intraurethral application, cannot be classified as a syringe and also does not apply liquid or gel, not confusing with present invention.

Other example of this, document US2008287879A1 refers to two devices for mixing medication at the time of use and an adapter for application. First and second syringes are filled with a lyophilized composition of vasoactive agents or a carrier gel. Furthermore, the solution includes a locking device coupling the syringes to facilitate the transfer of lyophilized composition of vasoactive agents and carrier gel between the syringes and a penile adapter for applying the therapeutic compounds for the treatment of erectile dysfunction of penis meatus. In this sense, two syringes coupled with an applicator demonstrate a different technology, not confusing with present invention.

Other example of this, document CN107921219A relates to a specific delivery method that describes a syringe device that can deliver therapeutic doses of a medicine in emergency situations where time is of the essence. The application method refers to an application using a needle, on the thigh. Therefore, this solution of CN107921219A does not meet the requirements for intraurethral application as it is intended for emergency situations using a needle for its application, without connector for intraurethral use, not confusing with present invention.

Other example of this, document CN110740771A refers to an automatic auto-injector with a needle capable of delivering one or more individual doses from a cartridge containing medication, for single or multiple use, frequently used for subcutaneous insulin injection and/or emergency injection. Therefore, this device does not meet the requirements for intraurethral application as it is intended for emergency situations, using needle for its application, without connector for intraurethral use, not confusing with present invention.

Other example of this, document CN108601517A refers to a 5 ml syringe connector of standard ISO594 for intraurethral instillation of pharmaceutical agents into the bladder without the use of a catheter. Although this solution allows intraurethral administration, the connector complies with a syringe manufacturing standard, but does not have a complete application device or a non-standard syringe, not confusing with present invention.

Thus, document WO2021026628A1 discloses an intraurethral medication applicator that has a receptacle divided into two interconnectable faces, where a non-standard syringe is fitted inside the receptacle. Holding the device between the fingers, the user removes the cap, holds the glans of the penis and applies the medication intraurethral through the extended nozzle of the syringe. Thus, the device of document WO2021026628A1 reveals an easy-to-use solution, allowing comfortable and discreet self-administration of medications into the urethra.

In addition to the aspect related to use, an intraurethral administration device needs to be safe both in its storage and in the correct quantity dispensed in the application. Knowing that the medication is at the site of action, the intraurethral route uses a lower dose of the medication than the oral or injectable route, providing the desired therapeutic effects without the systemic effect of conventional therapy with the same active ingredient, which facilitates the clearance of the drug. product and reduces toxicity.

Thus, from the literature searched, no documents were found anticipating or suggesting the teachings of the present invention, so that the solution proposed here has novelty and inventive step in relation to the prior art, complementing the previously published patent application in WO2021026628A1.

### Summary of the Invention

The present invention provides advantages in the intraurethral administration of medicines. Among the advantages and technical problems resolved, the following stand out: increased volumetric capacity for liquid or gel pharmaceutical compositions, a kit to accommodate the device, a plunger lock that prevents accidental activation of the plunger of a syringe, comfort in using the long nozzle of the syringe in the male urethral canal, ergonomics to adapt the user's fingers to the thicker device and versatility for application of different intraurethral therapies.

With this, present invention facilitates the administration of medication in urethral route, allowing reduction of side effects associated with the conventional treatments currently available orally or injectable; practicality of use both by the patient (self-administration) and by the healthcare professional; greater safety and comfort; reducing the amount of pharmaceutical active ingredient required for therapeutic action, expanding the therapeutic window and reducing toxicity. In this sense an intraurethral applicator of liquid or gel composition, provides easy self-administration, providing more comfort and reducing risks.

In one embodiment, the single dosage form comprises an intraurethral applicator device of liquid or gel composition that provides easy self-administration, providing more comfort and reducing risks.

The objects of the invention are device and its manufacturing process, where in the visible part, it has a graduation display (35) of syringe with increased volumetric capacity for intraurethral dosing of medicines, comprising: construction of a housing (30) comprising a flat surface (31) for receiving a plunger (10) and on the opposite side a flat surface (32) for receiving an extended syringe nozzle (40). The increased volume capacity comprises a domed shape arranged between the flat surface (31) and the opposite flat surface (32) where the syringe within the housing (30) is apparent and shows the graduation display (35).

Therefore, another object of the present invention is a kit for accommodate the device and a plunger lock that prevents accidental activation of the plunger of a syringe. Furthermore, it is another object of the present invention a process for manufacturing a device for intraurethral administration comprising steps of: construction of a housing (30) with a planar surface (31) for receiving a plunger (10) and an opposite planar surface (32) for fitting an extended syringe nozzle (40) and a cover (20); and manufacturing a non-standard syringe provided with a graduation display (35) and the extended syringe nozzle (40), being disposed inside the housing (30) with an increased volume capacity comprising a domed shape disposed between the planar surface (31) and the opposite planar surface (32).

These and other features of the present patent application will be described in detail below.

### Brief Description of Drawings

The following figures are shown:
Figure 1 shows a perspective view of one embodiment of applicator device that has a plunger (10), a cover (20) and a housing (30).
Figure 2 shows a perspective view of the same applicator device of figure 1 another projection that shows roughness (33) positioned on a wall of the housing (30).
Figure 3 shows a front view of the applicator device of figure 1, wherein an opening in the housing (30) provides a view of graduation display (35) of the syringe accommodated inside the housing (30).
Figure 4 shows a opposite view of front view the applicator device of figure 1.
Figure 5 shows a side view of the applicator device of figure 1, wherein a user activates a rod (12) of the plunger (10) associated to a planar surface (31) of the housing (30).
Figure 6 shows a side view of the applicator device of figure 1, wherein a cover (20) protects an extended syringe nozzle (40) of the device.
Figure 7 shows a top view of the applicator device of figure 1, with a plunger lock (37).
Figure 8 shows a side view of the applicator device of figure 1, removing the plunger lock (37) and the cover (20), and showing the extended syringe nozzle (40).
Figure 9 shows a rear view of the applicator device of figure 1, with the plunger lock (37). Also, wherein the cover (20) is removed showing the extended syringe nozzle (40).
Figure 10 shows a side view of the applicator device of figure 1, the tip (40) associated to an opposite planar surface (32) of the housing (30).
Figure 11 shows a perspective view of one embodiment of applicator device.
Figure 12 shows a front view of the applicator device of figure 11.
Figure 13 shows a kit for accommodate the device.
Figure 14 shows a plunger lock (37) that prevents accidental activation of syringe plunger (10).

### Detailed Description of the Invention

The present invention solves several technical problems by a device for intraurethral medication dosage in liquid or gel comprising: vasodilators, anti-inflammatories, antibiotics, antiseptics, anesthetics, among other pharmaceutical formulations for intraurethral use in men.

Among the various advantages highlights: the reducing the amount of pharmaceutical active required for therapeutic action; reduction of side effects associated to conventional treatments; expanding the therapeutic window and reducing toxicity; practicality of use by both the patient (self-administration) and the health professional; greater safety and comfort.

For purposes of the present invention the pharmaceutical composition is not being claimed, only the device.

In the context of the present patent application, the device provides intraurethral administration of pharmaceutical composition in liquid or gel form.

Besides, present invention purposes an administration of medications in urethral route by device with means of a smooth and delicate structure.

Preferably, the coloring of the intraurethral applicator is associated with the medication to be administered, such as: Blue for modulators of erectile function; Orange for anti-inflammatories; Green for anesthetics; Pink for antiseptics; Violet for antibiotics, among other colors and can be presented as an individual disposable option or kits according to the form of administration.

Without limiting the scope of the present invention, a cylindrical syringe provided with a display graduation (35) is fixed inside the housing (30). The housing (30) is a bipartite body comprising an increased width relative to prior art applicators. This width of housing (30) corresponds to a distance between walls (34). As a direct result of the increase in that width, the housing (30) gains usable space for storing the cylindrical syringe and, consequently, the housing (30) providing greater volume capacity.

The bipartite body of the housing (30) is divided in two concave parts. Each concave part has a sheltering shape that promotes ergonomics for the device mounted. Each concave part has a wall (34) in a flat area.

In external surface of each wall (34) there are plurality of encrusted projections or roughness (33). The external surface of the wall (34) is associated to a determined distribution of projections (33) preventing slipping by means of the grip generated by the projections (33).

It is noted that these embodiments do not restrict the scope of the present invention.

The housing (30) with an increased volumetric capacity has a domed shape that fits between fingers of a user holding it. In an embodiment, without restriction of the invention, a domed shape of the housing (30) has surfaces with a predefined curvature until each surface reaches a flat area. The flat areas correspond to the areas for fitting the user's fingers and fixing the plunger (10), extended syringe nozzle (40) and a cover to the nozzle (40). In this way, the housing (30) has curved surfaces (36) arranged between a planar surface (31) for receiving the plunger (10) and an opposite planar surface (32) for fitting the extended syringe nozzle (40), in addition to a plunger lock (37).

The plunger (10) has flanges (11) connected with a rod (12). The rod (12) fits into the flat face (31) of the housing (30), as an involucre, allowing robust attachment and movement of the plunger (10).

In an embodiment, the housing (30) has two curved surfaces (36) that are positioned in opposition and associated between two walls (34) and between the planar surface (31) and the opposite planar surface (32). The construction of planar and curved surfaces of this embodiment forms the domed shape of the housing (30) as a shape of "C" fitting a hand to promote ergonomics of use.

In one further embodiment, planar surface (31) and opposite planar surface (32) of the housing (30) are parallel surfaces, so that the curved surfaces (36) together with the walls (34) close the housing (30) connecting to the planar surface (31) and opposite planar surface (32). The opposite planar surface (32) has a larger area than planar surface (31), since opposite planar surface (32) receives the extended syringe nozzle (40) and a cover (20), protecting the extended nozzle (40). The cover (20) is disposed along the extended nozzle (40) and attached to the opposite planar surface (32).

Moreover, graduation marks are printed on a surface of the syringe, forming the graduation display (35), wherein the housing (30) has a window for viewing the graduation display (35), wherein the graduation marks are aligned to the window of the housing (30).

One of the walls (34) of the housing (30) has the window for viewing contents of syringe, either before application to check the content available or during application with help of the graduation marks of graduation display (35). A portion of the wall (34) is cropped to view the contents. In addition, the window of the housing (30) is positioned closer to the opposite planar surface (32) than to the planar surface (31) of the housing (30). This position allows the contents of the syringe to be viewed during the application. The projections (33) occupy at least two symmetrical portions of the wall (34), permitting use by either right or left hand. In an embodiment, the window is positioned between the two portions with projections (33), allowing user to hold the device on either side of the housing (30) without stop viewing the graduation display (35).

For a more comfortable application that adapts to each individual, the extended syringe nozzle (40) is made of a polymer, which facilitates the insertion of the extended syringe nozzle (40) into male urethra and delivery of the formulation. In an embodiment, the polymer gives the extended syringe nozzle (40) flexibility for delivery of the formulation, wherein the delivery is faithful to the volume in the syringe due to the polymer's property of not absorbing the composition. In an embodiment, without limiting the scope of the invention, the extended syringe nozzle (40) is made of silicone. In an alternative embodiment, the extended syringe nozzle (40) is made of at least two materials, one of which is flexible and comfortable in a portion for contact with the urethra, the other of which does not absorb the composition.

Thus, present invention provides the device for intraurethral medication dosage with greater volumetric capacity than prior art applicators, so that increase in volume provides the device with versatility for different therapies and treatments. In an embodiment, without limiting the scope of the invention, the device of present invention reaches 0.70 cm³. In one embodiment, the graduation marks on the graduation display (35) range from 200 µL to 700 µL.

In addition, the entire device is disposable. In an embodiment the syringe and its extended syringe nozzle (40) are disposable while the housing (30) is reusable. When using the device, in one embodiment, a user needs to open a package that has a lock associated with the device and/or with the package, in order to prevent accidental activation of the device. In an embodiment, the device is associated to a use lock.

It is another object of the invention, a kit for intraurethral administration that comprises the device for intraurethral medication dosage associated to a use lock, wherein the use lock prevents accidental triggering the device. In addition, the lock protects the user against tampering with the packaging or against improper reuse.

The use lock prevents accidental triggering the device. In an embodiment, the lock is a plunger lock (37) attached to the rod (12) of the plunger (10), blocking any movement of the plunger (10).

In this way, it is another object of the invention, the kit for accommodate the device against tampering with the packaging. In another modality, an individual plastic packaging, resistant and not very malleable.

The examples shown here are only intended to exemplify one of the countless ways of carrying out the invention, but without limiting its scope.

### Example 1 - Intraurethral device

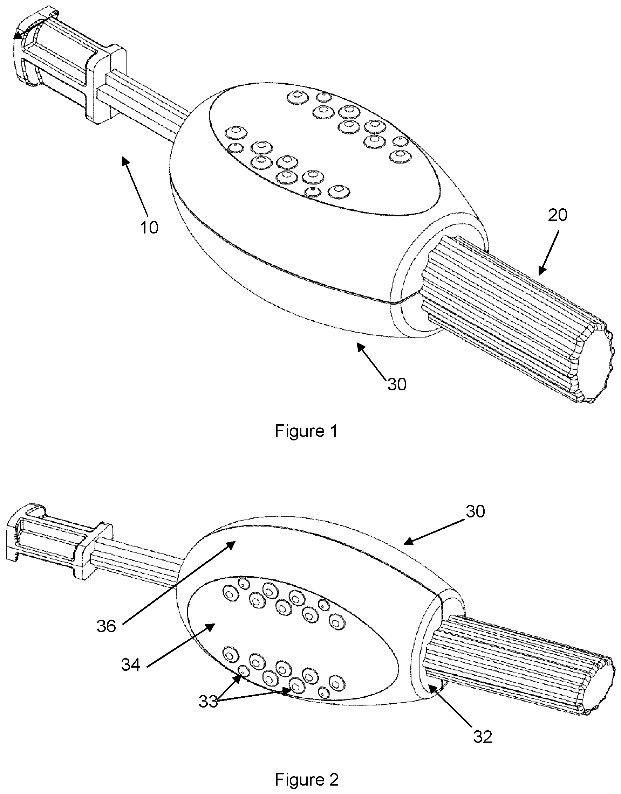

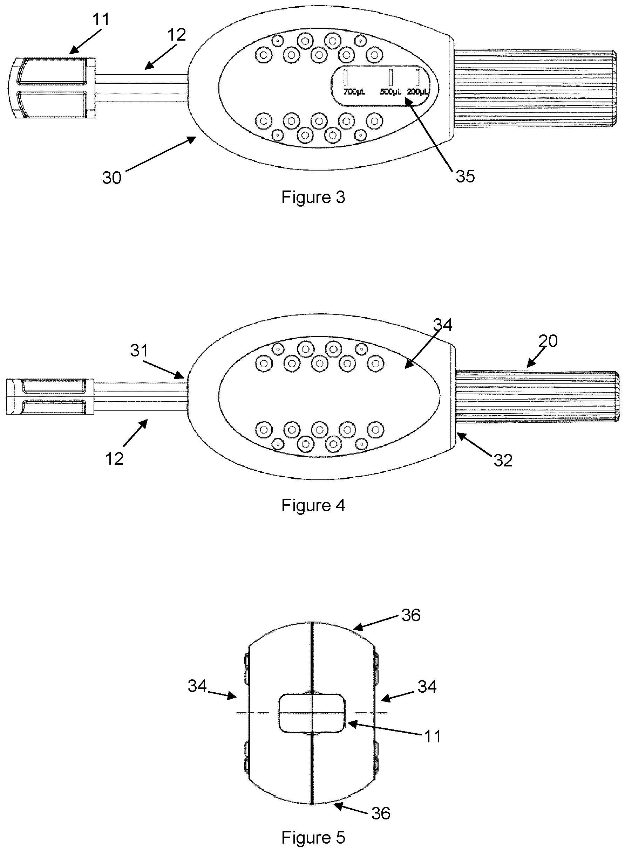

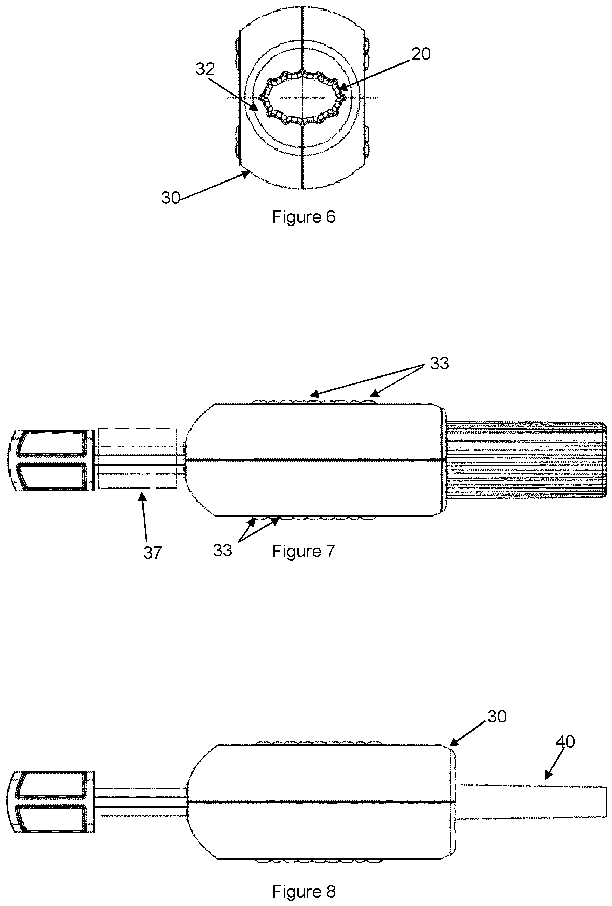

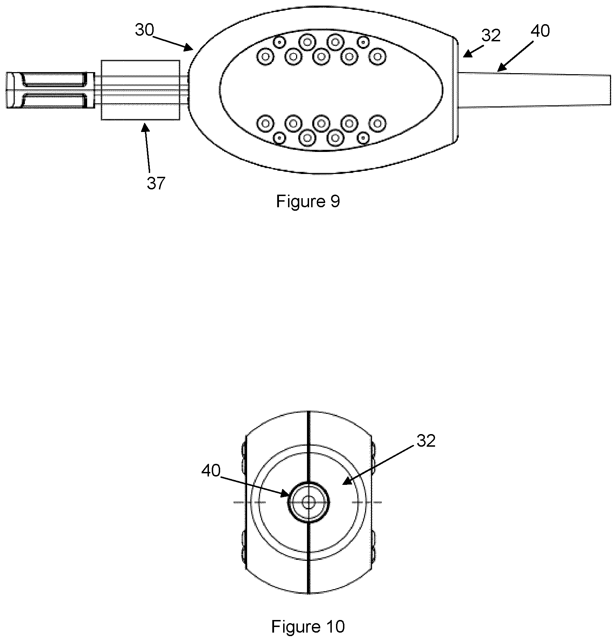

This embodiment of the present invention is described below with reference to the figures 1 to 10, which show an intraurethral device, without difficulty in handling. The device contains an involucre (30) that encloses a reservoir with a pharmaceutical compound.

Although the present invention increases the volumetric capacity of the reservoir, the stored volume is equal or less than 700 µL. For this, the set of the syringe is manufactured to comply with that smaller volume than conventional syringes. Thus, the present invention reduces the loss of the pharmaceutical compound stored, aiming for reliable application with the right amount by means of the extended syringe nozzle (40) impermeable to the pharmaceutical compound.

### Example 2 - Applicator device for men

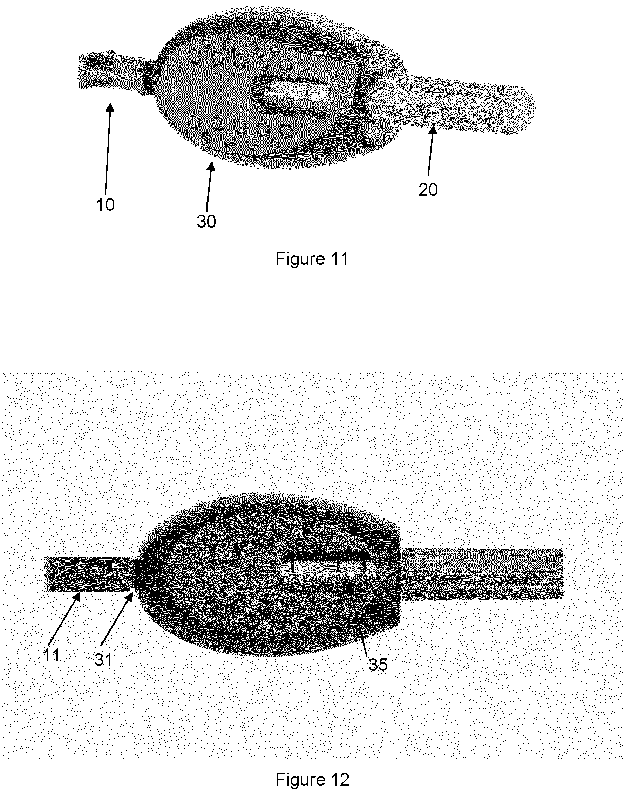

This embodiment of the present invention is described below with reference to the figures 11 and 12.

Figure 11 shows a representation of embodiment of an applicator device, wherein the involucre (30) is closed with a reservoir positioned internally and has a window aligned with a graduation display (35) of the reservoir, as shown in figure 12.

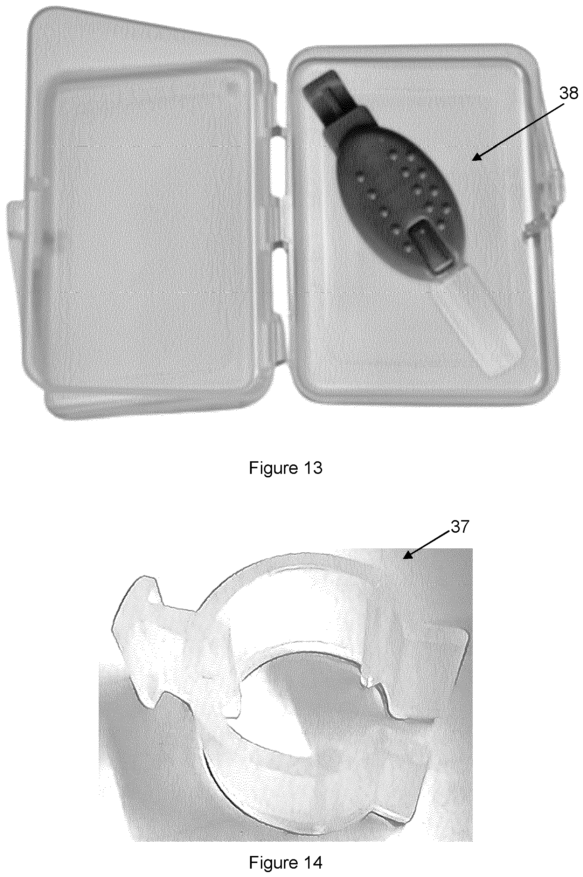

Without limiting the scope of the invention, figure 13 shows an exemplification of an applicator device (38) that is protected by a package which has a plunger lock (37), forming a kit to accommodate the device (38). The package has a shape that holds the device in position by involving the device, blocking movement of both the plunger (10) and a cover (20) covering the extended syringe nozzle (40). Thus, the applicator device (38) assembled is ready for use after opening the package and removing the cover (20) and the plunger lock (37). For this example, figure 14 shows a plunger lock (37) that has a peripherical wall in a "C" shape to attach the rod (12) of plunger (10). Each end of the peripherical wall of plunger lock (37) has a flap to maintain the rod (12) fixed, blocking movement of the plunger (10) to avoid accidental activation of the device. Also, the peripherical wall of the plunger lock (37) has a protuberance to detach it from the rod (12).

The reservoir is pre-filled with a single dose of pharmaceutical composition in liquid or gel for application in the urethra.

The user does not handle needles or sharp materials, since the extended syringe nozzle (40) is syringe part, penetrating 1 to 2 cm at the urethra of the penis to provide administration pharmaceutical composition.

Still, it is noted that the user applies the pharmaceutical composition in a simplified way by activating the plunger (10), bringing the flange (11) closer to the entry flat face (31) with good grip of the device, from the domed perimeter and the concavities of the curved faces (36), in addition to the protrusions (33) creating a zone of greater friction, as shown the figures 11 and 12 in comparison to the previous figures.

After pressing the plunger and administering the pharmaceutical composition into the urethra, the user can discard the set (single use - disposable) or can change the inner reservoir (multiple use - set kit discarded later).

The single dosage form of the invention is suitable for anti-inflammatories, antiseptics, antibiotics, anesthetics, among other applications to conditions that can receive local application.

Those skilled in the art will appreciate the knowledge presented here and will be able to reproduce the invention in the forms presented and in other variants and alternatives, covered by the scope of the following claims.

## Claims

1. Device for intraurethral administration comprising a housing (30) that provides a view of contents of a syringe by a graduation display (35) of the syringe associated between plunger (10) and extended syringe nozzle (40) **characterized in that** the housing (30) with an increased volumetric capacity comprises a domed shape disposed between a planar surface (31) for receiving the plunger (10) and an opposite planar surface (32) for fitting the extended syringe nozzle (40).

2. Device for intraurethral administration according to claim 1, **characterized in that** the housing (30) comprises at least a curved surface (36) associated between walls (34) and between the planar surface (31) and the opposite planar surface (32), forming the domed shape of the housing (30).

3. Device for intraurethral administration according to claim 2, **characterized in that** the housing (30) is a bipartite body comprising an increased width corresponding to a distance between the walls (34), providing the increased volumetric capacity.

4. Device for intraurethral administration according to claim 2, **characterized in that** the wall (34) comprises a plurality of projections with different sizes and/or roughness (33).

5. Device for intraurethral administration according to claim 1, **characterized in that** the housing (30) comprises at least a window for viewing the graduation display (35) of the syringe.

6. Device for intraurethral administration according to claim 5, **characterized in that** the window of the housing (30) is positioned closer to the opposite planar surface (32) than to the planar surface (31) of the housing (30).

7. Device for intraurethral administration according to claim 1, **characterized** for comprising a cover (20) being disposed along the extended syringe nozzle (40) when attached to the opposite planar surface (32) of the housing (30).

8. Device for intraurethral administration according to claim 4, **characterized in that** the projections (33) occupy at least two symmetric portions of the wall (34).

9. Device for intraurethral administration according to claim 1, **characterized** for comprising a plunger lock (37) blocking activation of the device.

10. Kit for intraurethral administration **characterized** for being tamper-proof by means of a package accommodating a device for intraurethral administration, wherein a plunger lock (37) prevents accidental triggering of the device.

11. Process for manufacturing a device for intraurethral administration **characterized** for comprising steps of:
a. construction of a housing (30) with a planar surface (31) for receiving a plunger (10) and an opposite planar surface (32) for fitting an extended syringe nozzle (40) and a cover (20); and
b. manufacturing a non-standard syringe provided with a graduation display (35) and the extended syringe nozzle (40), being disposed inside the housing (30) with an increased volume capacity comprising a domed shape disposed between the planar surface (31) and the opposite planar surface (32).

12. Process according to claim 11, **characterized** for comprising further steps of:
a. manufacturing a kit to accommodate the device, wherein the kit is tamper-proof by means of a package and/or an individual plastic package; and
b. manufacturing encrusted projections and/or roughness (33) on an external surface of the housing (30).
